# EUROPEAN PATENT APPLICATION

(11) **EP 4 378 942 A1**
(43) Date of publication of application: **05.06.2024**
(21) Application number: 22211191.6
(22) Date of filing: 02.12.2022
(51) Int. Cl.: C07D 471/04, A61P 11/00, A61P 11/06, A61P 17/00, A61P 17/06, A61P 27/02, A61P 29/00, A61P 31/12, A61P 37/00, A61K 31/519

(54) **CRYSTAL FORM OF A PDE3/4 INHIBITOR**

(71) Applicant: Sandoz AG, 4056 Basel (CH)
(72) Inventor: The designation of the inventor has not yet been filed
(74) Representative: Kluschanzoff, Harald

(57) **Abstract**

The present invention relates to a novel crystal of ensifentrine free base and to a process for its preparation. Furthermore, the invention relates to a pharmaceutical composition comprising said ensifentrine free base crystal, at least one pharmaceutically acceptable excipient. The pharmaceutical composition of the present invention can be used as a medicament, in particular for the treatment and/or prevention of asthma, chronic obstructive pulmonary disease (COPD), cystic fibrosis and symptoms of a COVID-19 infection.

## Description

### FIELD OF THE INVENTION

The present invention relates to a crystal of ensifentrine free base and to a process for its preparation. Furthermore, the invention relates to a pharmaceutical composition comprising said Ensifentrine free base crystal, at least one pharmaceutically acceptable excipient. The crystal and the pharmaceutical composition of the present invention can be use in the treatment or prevention of a disease or condition selected from asthma, allergic asthma, hay fever, allergic rhinitis, bronchitis, emphysema, bronchiectasis, chronic obstructive pulmonary disease (COPD), adult respiratory distress syndrome (ARDS), steroid resistant asthma, severe asthma, paediatric asthma, cystic fibrosis, lung fibrosis, pulmonary fibrosis, interstitial lung disease, skin disorders, atopic dermatitis, psoriasis, ocular inflammation, cerebral ischaemia, inflammatory diseases, auto-immune diseases and symptoms of a COVID-19 infection.

### BACKGROUND OF THE INVENTION

Ensifentrine is a potent and selective inhibitor of phosphodiesterases 3 and 4 (PDE3/4). PDE3 regulates cyclic adenosine monophosphate (cAMP) and cyclic guanosine monophosphate (cGMP) concentrations in airway smooth muscle cells. Not surprisingly, inhibition of PDE3 was found to result in airway smooth muscle relaxation. PDE4 regulates cAMP concentrations and was found to be involved in activation of inflammatory cells, whereas PDE4 inhibitors show potent anti-inflammatory activity. Simultaneous inhibition of PDE3 and PDE4 can mediate both anti-inflammatory and bronchodilator activity in additive or synergistic manner.

Ensifentrine is chemically also designated (9,10-dimethoxy-2-(2,4,6-trimethylphenylimino)-3-(N-carbamoyl-2-aminoethyl)-3,4,6,7-tetrahydro-2H-pyrimido[6,1-a]isoquinolin-4-one) and can be represented by the chemical structure as depicted in formula A:

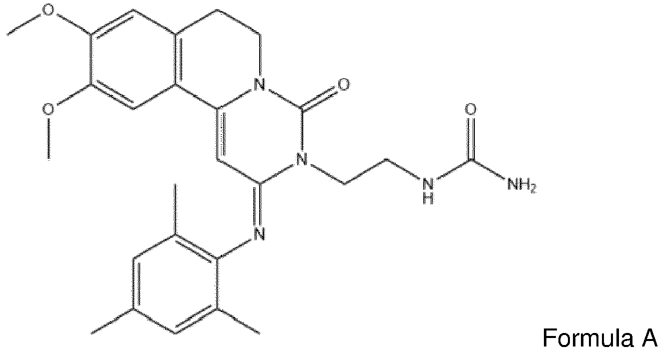

The compound Ensifentrine was first disclosed in WO 2000/058308 A1 as a yellow solid. A polymorph screen disclosed in WO 2012/020016 A1 led to the identification of crystalline ensifentrine Form I. In WO 2016/128742 discloses ensifentrine acid addition salts with ethane-1,2-disulfonic acid, ethanesulfonic acid, methanesulfonic acid, benzenesulfonic acid, p-toluenesulfonic acid, hydrochloric acid hydrobromic acid, phosphoric acid or sulfuric acid.

Different solid-state forms of an active pharmaceutical ingredient (API) often possess different physical and chemical properties such as but not limited to dissolution rate, solubility, chemical stability, physical stability, hygroscopicity, melting point, morphology, flowability, bulk density and compressibility. Apart from conventional solid-state forms of an API, such as polymorphs, pseudopolymorphs (hydrates and solvates) and salts, pharmaceutical co-crystals open up further opportunities for customizing the physicochemical properties of APIs with a process or clinical need. For example, they can be tailored to enhance drug product bioavailability and stability and to enhance the processability of APIs during drug product manufacture.

It is thus an objective of the present invention to provide an improved solid-state form of ensifentrine, in particular a crystal of ensifentrine, which is physically stable against temperature stress. It is a further objective of the present invention to provide an improved solid-state form of Ensifentrine, in particular a crystal of ensifentrine, which is characterized by improved dissolution/solubility and/or characterized by improved powder characteristics such as flowability, bulk density and compressibility.

### SUMMARY OF THE INVENTION

The invention solves one or more of the above defined objectives by providing a novel pharmaceutical crystal of ensifentrine free base (Form A). The crystal of the present invention possesses one or more improved physicochemical properties selected from dissolution rate, solubility, chemical stability, physical stability, hygroscopicity, melting point, morphology, flowability, bulk density and compressibility.

### Abbreviations

- PXRD: powder X-ray diffractogram
- FTIR: Fourier transform infrared
- ATR: attenuated total reflection
- DSC: differential scanning calorimetry
- TGA: thermogravimetric analysis
- NMR: nuclear magnetic resonance
- RT: room temperature
- RH: relative humidity
- API: active pharmaceutical ingredient

### Definitions

As used herein the term "room temperature" refers to a temperature in the range of from 20 to 30 °C.

The term "Ensifentrine" as used herein refers to (9,10-dimethoxy-2-(2,4,6-trimethylphenylimino)-3-(N-carbamoyl-2-aminoethyl)-3,4,6,7-tetrahydro-2H-pyrimido[6,1-a]isoquinolin-4-one) according to the chemical structure depicted in formula A disclosed herein above.

The term "ensifentrine form I" as used herein, refers to the crystalline form I of ensifentrine, which is disclosed in WO 2012/020016 A1. Form A of Ensifentrine can be characterized by having a powder X-ray diffractogram comprising reflections at 2-Theta angles of (5.7 ± 0.2)°, (9.8 ± 0.2)° and (20.0 ± 0.2)°, when measured at a temperature in the range of from 20 to 30 °C with Cu-Kalpha_{1,2} radiation having a wavelength of 0.15419 nm.

As used herein, the term "measured at a temperature in the range of from 20 to 30 °C" refers to a measurement under standard conditions. Typically, standard conditions mean a temperature in the range of from 20 to 30 °C, i.e. at room temperature. Standard conditions can mean a temperature of about 22 °C. Typically, standard conditions can additionally mean a measurement under 20-50% relative humidity.

The term "reflection" with regard to powder X-ray diffraction as used herein, means peaks in an X-ray diffractogram, which are caused at certain diffraction angles (Bragg angles) by constructive interference from X-rays scattered by parallel planes of atoms in solid material, which are distributed in an ordered and repetitive pattern in a long-range positional order. Such a solid material is classified as crystalline material, whereas amorphous material is defined as solid material, which lacks long-range order and only displays short-range order, thus resulting in broad scattering. According to literature, long-range order e.g. extends over approximately 100 to 1000 atoms, whereas short-range order is over a few atoms only (see *"*Fundamentals of Powder Diffraction and Structural Characterization of Materials" by Vitalij K. Pecharsky and Peter Y. Zavalij, Kluwer Academic Publishers, 2003, page 3).

The term "essentially the same" with reference to powder X-ray diffraction means that variabilities in reflection positions and relative intensities of the reflections are to be taken into account. For example, a typical precision of the 2-Theta values is in the range of ± 0.2° 2-Theta, preferably in the range of ± 0.1° 2-Theta. Thus, a reflection that usually appears at 5.7° 2-Theta for example can appear between 5.5° and 5.9° 2-Theta, preferably between 5.6° and 5.8° 2-Theta on most X-ray diffractometers under standard conditions. Furthermore, one skilled in the art will appreciate that relative reflection intensities will show inter-apparatus variability as well as variability due to degree of crystallinity, preferred orientation, sample preparation and other factors known to those skilled in the art and should be taken as qualitative measure only.

The term "essentially the same" with reference to Fourier transform infrared spectroscopy means that variabilities in peak positions and relative intensities of the peaks are to be taken into account. For example, a typical precision of the wavenumber values is in the range of ± 2 cm⁻¹. Thus, a peak at 3468 cm⁻¹ for example can appear in the range of from 3470 to 3466 cm⁻¹ on most infrared spectrometers under standard conditions. Differences in relative intensities are typically smaller compared to X-ray diffraction. However, one skilled in the art will appreciate that small differences in peak intensities due to degree of crystallinity, sample preparation and other factors can also occur in infrared spectroscopy. Relative peak intensities should therefore be taken as qualitative measure only.

The term "solid-state form" as used herein refers to any crystalline and/or amorphous phase of a compound. Crystalline phases include anhydrous/non-solvated forms of a compound and their polymorphs, hydrates and solvates of a compound and their polymorphs, salts and co-crystals of a compound and any mixtures thereof.

As used herein, the term "essentially free of any other solid-state form" with reference to the composition comprising the ensifentrine Form A crystal of the present invention, means that the ensifentrine Form A crystal contains at most 20 weight%, preferably at most 10 weight%, more preferably at most 5 weight%, 4 weight%, 3 weight%, 2 weight% or 1 weight% of any other solid-state form of ensifentrine, in particular ensifentrine Form A, based on the weight of the composition.

The terms "anhydrous" or "anhydrate" as used herein refer to a crystalline solid where no water is cooperated in or accommodated by the crystal structure. Anhydrous forms may still contain residual water, which is not part of the crystal structure but may be adsorbed on the surface or absorbed in disordered regions of the crystal. Typically, an anhydrous form does not contain more than 1.0 weight%, preferably not more than 0.5 weight% and most preferably not more than 0.3 weight%, 0.2 weight% or 0.1 weight% of water, based on the weight of the crystalline form. The water content can be determined by Karl-Fischer Coulometry and/or by thermogravimetric analysis (TGA), e.g. by determining the mass loss in the range of from 25 to 180 °C, 190 °C or 200 °C at a heating rate of 10 K/min.

The term "non-solvated" as used herein, when talking about a crystalline solid indicates that no organic solvent is cooperated in or accommodated by the crystal structure. Non-solvated forms may still contain residual organic solvents, which are not part of the crystal structure but may be adsorbed on the surface or absorbed in disordered regions of the crystal. Typically, a non-solvated form does not contain more than 1.0 weight%, preferably not more than 0.5 weight%, and most preferably not more than 0.3 weight%, 0.2 weight% or 0.1 weight% of organic solvents, based on the weight of the crystalline form. The organic solvent content can be determined by thermogravimetric analysis (TGA), e.g. by determining the mass loss in the range of from 25 to 300 °C at a heating rate of 10 K/min or by ¹H-NMR.

The ensifentrine Form A crystal may be referred to herein as being characterized by a powder X-ray diffractogram, a Fourier transform infrared spectrum and/or a "as shown in" a figure. The person skilled in the art understands that factors such as variations in instrument type, response and variations in sample directionality, sample concentration, sample purity, sample history and sample preparation may lead to variations, for example relating to the exact reflection or peak positions and intensities. However, a comparison of the graphical data in the figures herein with the graphical data generated for an unknown physical form and the confirmation that two sets of graphical data relate to the same crystal form is well within the knowledge of a person skilled in the art.

As used herein, the term "mother liquor" refers to the solution remaining after crystallization of a solid from said solution.

A "predetermined amount" as used herein with regard to ensifentrine Form A crystal of the present invention refers to the initial amount of the ensifentrine Form A crystal used for the preparation of a pharmaceutical composition having a desired dosage strength of ensifentrine.

As used herein, the term "effective amount" in conjunction with the ensifentrine Form A crystal of the present invention encompasses an amount of the the ensifentrine Form A crystal which causes the desired therapeutic or prophylactic effect.

As used herein, the term "about" means within a statistically meaningful range of a value. Such a range can be within an order of magnitude, typically within 10%, more typically within 5%, even more typically within 1% and most typically within 0.1% of the indicated value or range. Sometimes, such a range can lie within the experimental error, typical of standard methods used for the measurement and/or determination of a given value or range.

The term "pharmaceutically acceptable excipient" as used herein refers to substances, which do not show a significant pharmacological activity at the given dose and that are added to a pharmaceutical composition in addition to the active pharmaceutical ingredient. Excipients may take the function of vehicle, diluent, release agent, surfactant, tonic adjuster, buffer, disintegrating agent, dissolution modifying agent, absorption enhancer, stabilizer or a manufacturing aid among others. Excipients may include fillers, diluents, surfactant, tonic adjuster, buffer, binders, disintegrants, lubricants and glidants.

The terms "filler" or "diluent" as used herein refer to substances that are used to dilute the active pharmaceutical ingredient prior to delivery. Diluents and fillers can also serve as stabilizers.

As used herein the term "binder" refers to substances which bind the active pharmaceutical ingredient and pharmaceutically acceptable excipient together to maintain cohesive and discrete portions.

The terms "disintegrant" or "disintegrating agent" as used herein refers to substances which, upon addition to a solid pharmaceutical composition, facilitate its break-up or disintegration after administration and permits the release of the active pharmaceutical ingredient as efficiently as possible to allow for its rapid dissolution.

The term "lubricant" as used herein refers to substances which are added to a powder blend to prevent the compacted powder mass from sticking to the equipment during tableting or encapsulation process. They aid the ejection of the tablet from the dies and can improve powder flow.

The term "glidant" as used herein refers to substances which are used for tablet and capsule formulations in order to improve flow properties during tablet compression and to produce an anti-caking effect.

### BRIEF DESCRIPTION OF THE FIGURES

**Figure 1****:** illustrates a representative PXRD of the ensifentrine Form A crystal according to the present invention. The x-axis shows the scattering angle in °2-Theta, the y-axis shows the intensity of the scattered X-ray beam in counts of detected photons.
**Figure 2****:** illustrates a representative FTIR spectrum of the ensifentrine Form A crystal according to the present invention. The x-axis shows the wavenumbers in cm⁻¹, the y-axis shows the relative intensity in percent transmittance.

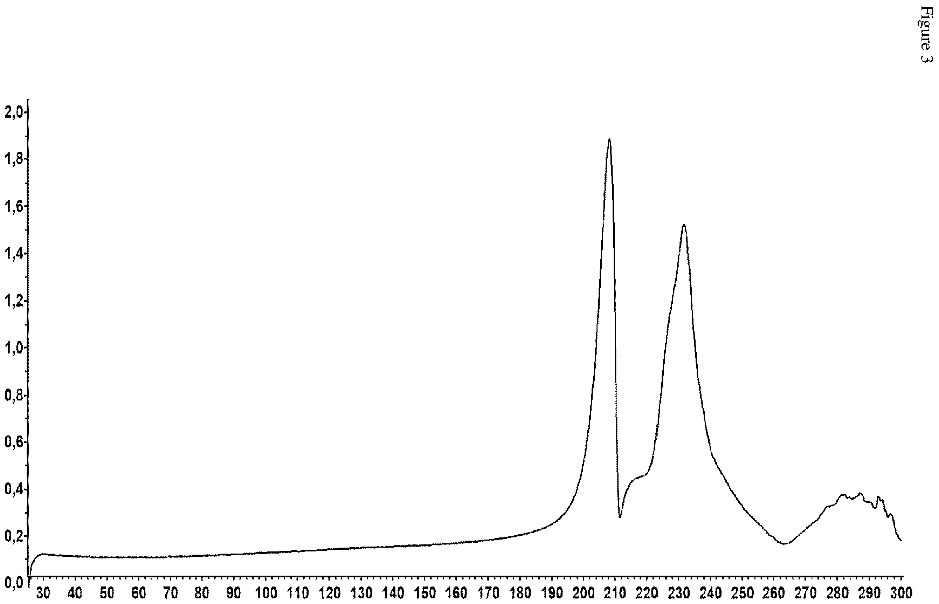

**Figure 3****:** illustrates a representative DSC curve of the ensifentrine Form A crystal according to the present invention. The x-axis shows the temperature in degree Celsius (°C), the y-axis shows the heat flow rate in Watt per gram (W/g) with endothermic peaks going up.

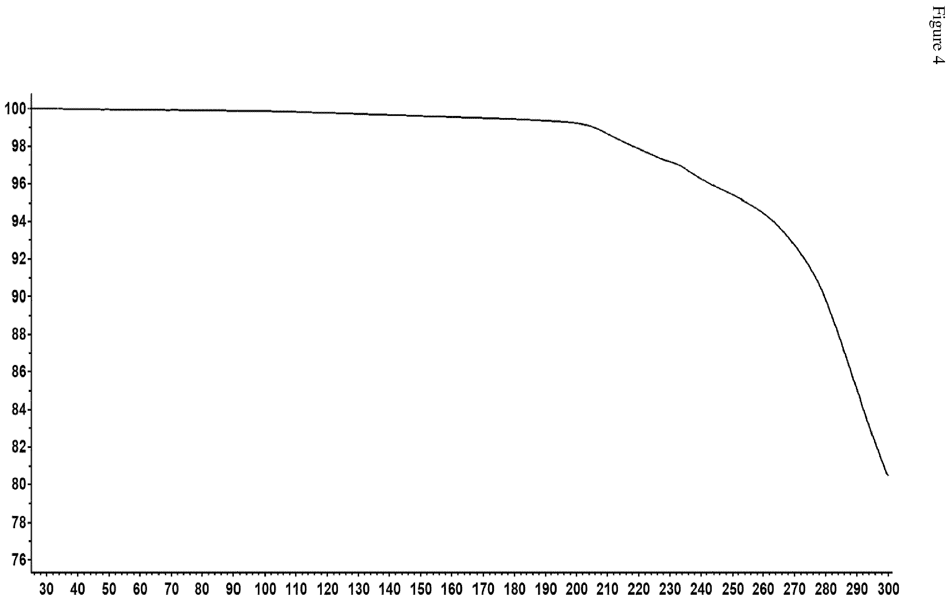

**Figure 4****:** illustrates a representative TGA curve of the Ensifentrine Form A crystal according to the present invention. The x-axis shows the temperature in degree Celsius (°C), the y-axis shows the mass (loss) of the sample in weight percent (weight%).

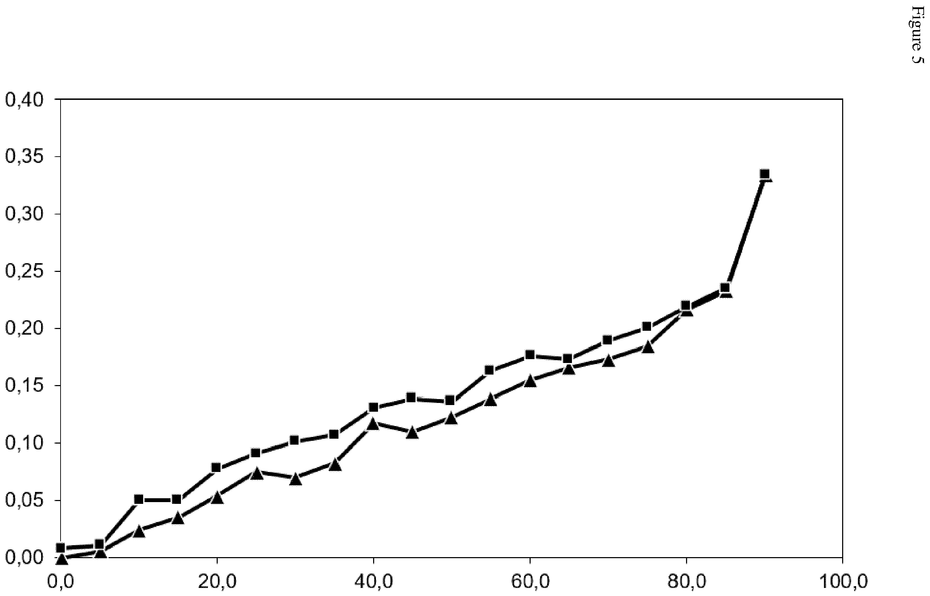

**Figure 5****:** illustrates a representative GMS isotherms of ensifentrine Form A crystal of the present invention in the range of from 0 to 90% relative humidity. The x-axis displays the relative humidity in percent (%) measured at a temperature of (25.0 ± 0.1)°C, the y-axis displays the equilibrium mass change in weight percent (w-%). Sample weight at 0% relative humidity at the start of the sorption curve is used as reference weight. Sorption curve points are displayed as triangles, desorption curve points as squares.

### DETAILED DESCRIPTION OF THE INVENTION

The ensifentrine Form A crystal of the present invention is physically stable toward temperature stress e.g. it shows no thermal events in a DSC experiment up to a temperature of approximately 200°C. Moreover, a TGA experiment performed with the crystalline form of the present invention revealed a mass loss of about 0.5% or less up to a temperature of 200°C. Above 200 °C a phase transition and a mass loss of about 5% until melting, which indicates the presence of a solvated solid-state form, which was finally proved by PXRD. Furthermore, preliminary stability testing at 40°C/75%rh, 30°C/65%rh, 25°C/60%rh and RT/97%rh did not result in changes of the PXRD spectrum and TGA curve after one week. In addition, the ensifentrine Form A crystal of the present invention shows advantageous dissolution behavioiur, good chemical and physical stability and is characterized by excellent powder properties such as good flowability, high bulk density and good compressibility. All in all, these favorable attributes allow for a robust formulation and ensure a reliable safety and efficacy profile of a drug product containing the ensifentrine Form A crystal of the present invention during the whole shelf-life of the product.

The ensifentrine Form A crystal of the present invention may be characterized by analytical methods well known in the field of the pharmaceutical industry for characterizing crystalline solids. Such methods comprise but are not limited to powder and single X-ray diffraction, Fourier transform and, DSC, TGA and GMS. The crystal of the present invention may be characterized by one of the aforementioned analytical methods or by combining two or more of them. In particular, the crystal of the present invention may be characterized by any one of the following embodiments or by combining two or more of the following embodiments.

In one embodiment the invention relates to a crystal of ensifentrine Form A characterized by having a PXRD comprising reflections at 2-Theta angles of:
(5.7 ± 0.2)°, (9.8 ± 0.2)° and (20.0 ± 0.2)°; or
(5.7 ± 0.2)°, (9.8 ± 0.2)°, (20.0 ± 0.2)° and (23.3 ± 0.2)°; or
(5.7 ± 0.2)°, (9.8 ± 0.2)°, (13.7 ± 0.2)°, (20.0 ± 0.2)° and (23.3 ± 0.2)°; or
(5.7 ± 0.2)°, (9.8 ± 0.2)°, (13.7 ± 0.2)°, (20.0 ± 0.2)°, (23.3 ± 0.2)° and (25.3 ± 0.2)°; or
(5.7 ± 0.2)°, (9.8 ± 0.2)°, (13.7 ± 0.2)°, (14.2 ± 0.2)°, (20.0 ± 0.2)°, (23.3 ± 0.2)° and (25.3 ± 0.2)°; or
(5.7 ± 0.2)°, (6.7 ± 0.2)°, (9.8 ± 0.2)°, (13.7 ± 0.2)°, (14.2 ± 0.2)°, (20.0 ± 0.2)°, (23.3 ± 0.2)° and (25.3 ± 0.2)°; or
(5.7 ± 0.2)°, (6.7 ± 0.2)°, (9.8 ± 0.2)°, (13.7 ± 0.2)°, (14.2 ± 0.2)°, (16.4 ± 0.2)°, (20.0 ± 0.2)°, (23.3 ± 0.2)° and (25.3 ± 0.2)°; or
(5.7 ± 0.2)°, (6.7 ± 0.2)°, (9.8 ± 0.2)°, (13.7 ± 0.2)°, (14.2 ± 0.2)°, (16.4 ± 0.2)°, (18.7 ± 0.2)°, (20.0 ± 0.2)°, (23.3 ± 0.2)° and (25.3 ± 0.2)°,
when measured at RT with Cu-Kalpha_{1,2} radiation having a wavelength of 0.15419 nm.

In a further embodiment the invention relates to a crystal of ensifentrine Form A characterized by having a PXRD comprising reflections at 2-Theta angles of:
(5.7 ± 0.1)°, (9.8 ± 0.1)° and (20.0 ± 0.1)°; or
(5.7 ± 0.1)°, (9.8 ± 0.1)°, (20.0 ± 0.1)° and (23.3 ± 0.1)°; or
(5.7 ± 0.1)°, (9.8 ± 0.1)°, (13.7 ± 0.1)°, (20.0 ± 0.1)° and (23.3 ± 0.1)°; or
(5.7 ± 0.1)°, (9.8 ± 0.1)°, (13.7 ± 0.1)°, (20.0 ± 0.1)°, (23.3 ± 0.1)° and (25.3 ± 0.1)°; or
(5.7 ± 0.1)°, (9.8 ± 0.1)°, (13.7 ± 0.1)°, (14.2 ± 0.1)°, (20.0 ± 0.1)°, (23.3 ± 0.1)° and (25.3 ± 0.1)°; or
(5.7 ± 0.1)°, (6.7 ± 0.1)°, (9.8 ± 0.1)°, (13.7 ± 0.1)°, (14.2 ± 0.1)°, (20.0 ± 0.1)°, (23.3 ± 0.1)° and (25.3 ± 0.1)°; or
(5.7 ± 0.1)°, (6.7 ± 0.1)°, (9.8 ± 0.1)°, (13.7 ± 0.1)°, (14.2 ± 0.1)°, (16.4 ± 0.1)°, (20.0 ± 0.1)°, (23.3 ± 0.1)° and (25.3 ± 0.1)°; or
(5.7 ±0.1)°, (6.7 ±0.1)°, (9.8 ±0.1)°, (13.7 ±0.1)°, (14.2 ±0.1)°, (16.4 ±0.1)°, (18.7 ± 0.1)°, (20.0 ± 0.1)°, (23.3 ± 0.1)° and (25.3 ± 0.1)°,
when measured at RT with Cu-Kalpha_{1,2} radiation having a wavelength of 0.15419 nm.

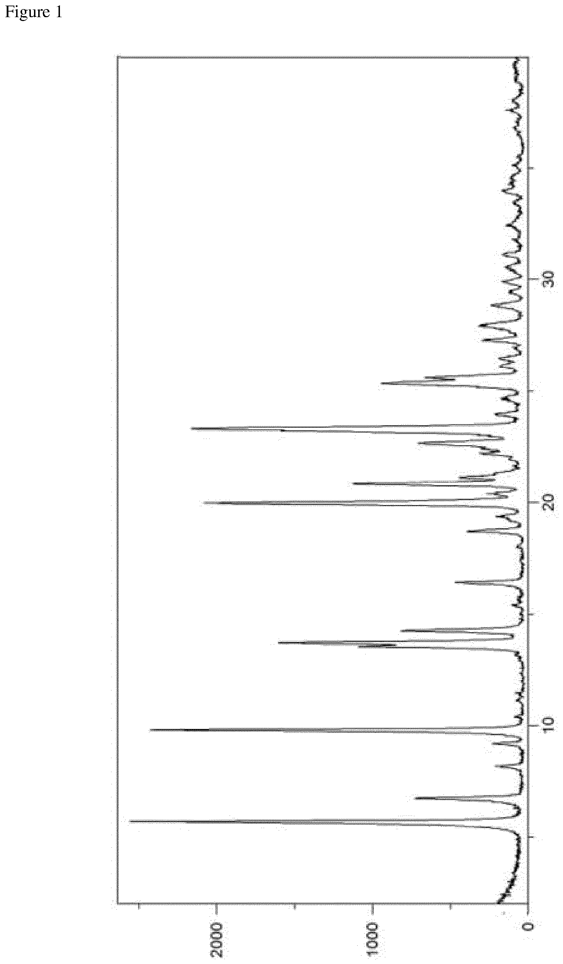

In yet another embodiment the invention relates to a crystal of ensifentrine Form A characterized by having a PXRD essentially the same as shown in figure 1 of the present invention, when measured at RT with Cu-Kalpha_{1,2} radiation having a wavelength of 0.15419 nm.

In a further embodiment, the present invention relates to a crystal of ensifentrine Form A, characterized by having an FTIR spectrum comprising peaks at wavenumbers of:
(3468 ± 2) cm⁻¹, (2931 ± 2) cm⁻¹ and (1644 ± 2) cm⁻¹ or;
(3468 ± 2) cm⁻¹, (3351 ± 2) cm⁻¹, (2931 ± 2) cm⁻¹ and (1644 ± 2) cm⁻¹ or;
(3468 ± 2) cm⁻¹, (3351 ± 2) cm⁻¹, (2931 ± 2) cm⁻¹, (1644 ± 2) cm⁻¹ and (1594 ± 2) cm⁻¹ or;
(3468 ± 2) cm⁻¹, (3351 ± 2) cm⁻¹, (2931 ± 2) cm⁻¹, (1644 ± 2) cm⁻¹ (1594 ± 2) cm⁻¹ and (1222 ± 2) cm⁻¹ or;
(3468 ± 2) cm⁻¹, (3351 ± 2) cm⁻¹, (2931 ± 2) cm⁻¹, (1644 ± 2) cm⁻¹, (1594 ± 2) cm⁻¹, (1512 ± 2) cm⁻¹, (1436 ± 2) cm⁻¹ and (1222 ± 2) cm⁻¹ or;
(3468 ± 2) cm⁻¹, (3351 ± 2) cm⁻¹, (2931 ± 2) cm⁻¹, (1644 ± 2) cm⁻¹, (1594 ± 2) cm⁻¹, (1512 ± 2) cm⁻¹, (1436 ± 2) cm⁻¹, (1222 ± 2) cm⁻¹ and (792 ± 2) cm⁻¹ or;
(3468 ± 2) cm⁻¹, (3351 ± 2) cm⁻¹, (2931 ± 2) cm⁻¹, (1644 ± 2) cm⁻¹, (1594 ± 2) cm⁻¹, (1512 ± 2) cm⁻¹, (1436 ± 2) cm⁻¹, (1222 ± 2) cm⁻¹, (993 ± 2) cm⁻¹ and (792 ± 2) cm⁻¹ or, when measured at RT with a diamond ATR cell.

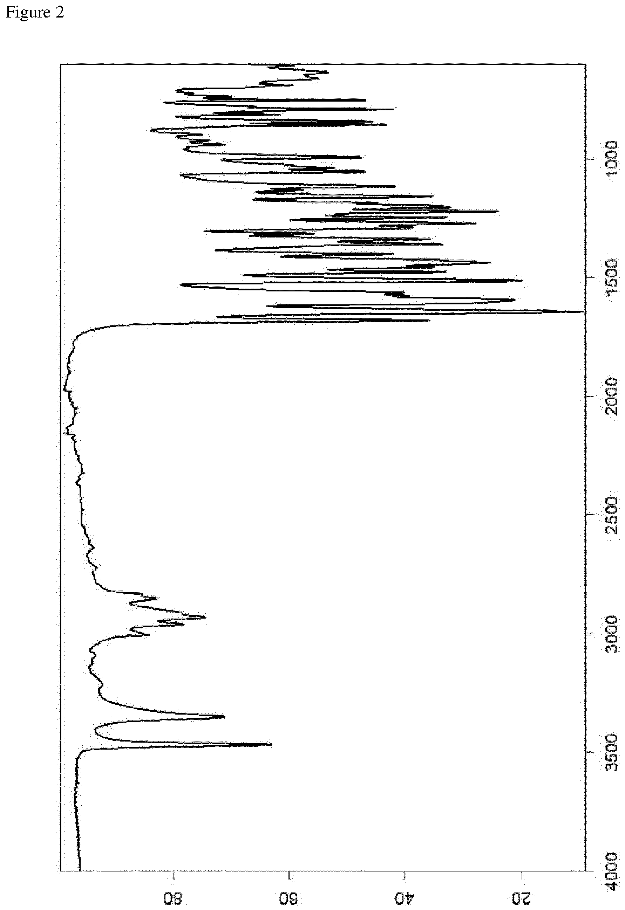

In yet another embodiment, the present invention relates to a crystalline form of ensifentrine free base, characterized by having a FTIR spectrum essentially the same as shown in figure 2 of the present invention, when measured at RT with a diamond ATR cell.

In one embodiment, the present invention relates to crystal of ensifentrine Form A characterized in that the crystal is solvated.

In another embodiment, the present invention relates to a crystal of ensifentrine Form A is a dichloromethane solvate.

In another embodiment, the present invention relates to a crystal of ensifentrine Form A, characterized by having a DSC curve comprising a first endothermic peak, having an onset temperature of (201 ±1)°C, and a second endothermic peak, preferably a second endothermic peak, having an onset temperature of (221 ±1)°C, when measured with DSC at a heating rate of 10 K/min.

In a further embodiment, the present invention relates to a crystal of ensifentrine Form A, characterized by having a DSC curve comprising a first endothermic peak, having a peak maximum temperature of (208 ± 1)°C and a second endothermic peak, having a peak maximum temperature of (231 ± 1)°C, when measured with DSC at a heating rate of 10 K/min.

In another embodiment, the present invention relates to a crystal of ensifentrine Form A, characterized by having a TGA curve showing a mass loss of 0.2 weight% or less, when heated from RT to 100 °C at a rate of 10 K/min.

In a further embodiment, the present invention relates to a crystal of ensifentrine Form A, characterized by having a TGA curve showing a mass loss of 1 weight% or less, when heated from RT to 200 °C at a rate of 10 K/min.

In yet a further embodiment, the present invention relates to a crystal of Ensifentrine Form A, characterized by having a TGA curve showing a mass loss of about 5 weight%, when heated from 170 °C to 260 °C at a rate of 10 K/min.

Thermal analyses such as DSC and TGA revealed that the crystal of ensifentrine Form A of the present invention is thermally highly stable e.g. does not undergo phase transformations or decomposition up to a temperature of 200 °C. Heating to 210 °C finally leads to a phase transformation into ensifentrine Form I first disclosed in WO 2012/020016 A1 as indicated by an PXRD. Independently, the TGA shows the phase transition occurring at a temperature above 200 °C is accompanied with a continous loss of solvent until melting of the sample.

Hence, the thermal stability of the ensifentrine Form A crystal is sufficient for use in a pharmaceutical composition and preparation of a medicament.

In a further embodiment, the present invention relates to ensifentrine Form Form A crystal characterized by showing a mass change of about 0.3 weight%, based on the weight of the crystalline form, when measured with GMS at a relative humidity in the range of from 0 to 90% and a temperature of (25 0 + 1.0)°C as outlined in figure 6. A PXRD measured after the GMS experiment corresponds with that of the original sample.

Hence, the stability under humid conditions of the ensifentrine Form A crystal is sufficient for use in a pharmaceutical composition and preparation of a medicament.

In another aspect, the present invention relates to a composition comprising the ensifentrine Form A crystal of the present invention as defined in any of the embodiments described above, said composition being essentially free of any other solid-state form of Ensifentrine. For example, a composition comprising the ensifentrine Form A crystal of the present invention comprises at most 20 weight%, preferably at most 10 weight%, more preferably at most 5 weight%, 4 weight%, 3 weight%, 2 weight% or 1 weight% of any other solid-state form of ensifentrine, based on the weight of the composition. Preferably, the any other solid-state form of Ensifentrine is form I of WO 2012/020016 A1. Form I of ensifentrine has a PXRD comprising amongst others characteristic reflections at 2-Theta angles of (10.1 ± 0.2)° and (12.9 ± 0.2)°, when measured at a temperature in the range of from 20 to 30 °C with Cu-Kalpha_{1,2} radiation having a wavelength of 0.15419 nm. Therefore, the absence of reflections at 2-Theta angles of (10.1 ± 0.2)° and (12.9 ± 0.2)° in the PXRD confirms the absence of Ensifentrine form I in the composition.

Hence, in a preferred embodiment, the present invention relates to a composition comprising the ensifentrine Form A crystal of the present invention as defined in any of the embodiments described above, said composition having a PXRD comprising no reflections at 2-Theta angles of (10.1 ± 0.2)° and (12.9 ± 0.2)°, when measured at a temperature in the range of from 20 to 30 °C with Cu-Kalpha_{1,2} radiation having a wavelength of 0.15419 nm.

In a further aspect, the present invention relates to a process for the preparation of the ensifentrine Form A crystal of the present invention or the composition comprising the ensifentrine Form A crystal as defined in any one of the aspects and their corresponding embodiments described above comprising:
(a) dissolving ensifentrine in a solvent mixture comprising methanol and dichloromethane;
(b) optionally, removing insoluble particles by filtration
(c) optionally, seeding the mixture obtained in (a or b) with ensifentrine Form A crystals according to the present invention;
(d) removing the solvent at reduced pressure
(e) optionally, drying the crystals.

Ensifentrine can for example be prepared according to procedures provided in example 1 of WO 2000/058308 A1 and example 17 of WO 2018/020249 A1. Amorphous ensifentrine which may be prepared according to the procedures disclosed in WO 2012/020016 A1 is used as starting material for the production of the ensifentrine Form A crystal of the present invention.

Optionally, ensifentrine Form A crystals may be added as seeds in order to promote crystallization and/or to control particle size distribution. The amount of seed crystals employed may range from about 1 to 20 weight%, preferably from about 1 to 10 weight% and most preferably from about 1 to 5 weight%, based on the weight of applied Ensifentrine starting material. Seed crystals may be prepared according to steps (a) to (b) of the above described procedure e.g. according to the procedure disclosed in example 1 of the present invention.

Optionally, in a further step the isolated crystals are washed with at least one aliphatic ether selected from the group consisting of diisopropyl ether, tert-butylmethyl ether and diethyl ether or any mixtures thereof. Preferably, diisopropyl ether and/or *tert-*butylmethyl ether are used.

The obtained crystals may then optionally be dried. Drying may be performed at a temperature in the range of from about 20 to 80 °C, preferably in the range of from about 20 to 50 °C. Drying may be performed for a period in the range of from about 1 to 72 hours, preferably of from about 2 to 48 hours, more preferably of from about 4 to 24 hours and most preferably of from about 6 to 18 hours. Drying may be performed at ambient pressure and/ or under reduced pressure. Preferably, drying is performed at a pressure of about 100 mbar or less, more preferably of about 50 mbar or less and most preferably of about 30 mbar or less, for example a vacuum of about 25 mbar is applied for drying.

### Pharmaceutical compositions and medical use

In a further aspect, the present invention relates to the use of crystalline ensifentrine Form A for the preparation of a pharmaceutical composition.

In another aspect, the present invention relates to particles comprising crystalline ensifentrine Form A and optionally at least one pharmaceutically acceptable excipient, having a particle size distribution with a Dv50 value of from about 0.25 µm to about 5 µm. These particles may be produced by a particle size controlled production process or any pharmaceutically acceptable size reduction process.

Preferably, the amount of crystalline ensifentrine Form A in the particles may range from about 50 to 100 weight%, preferably from about 80 to 100 weight% and most preferably 100% weight%.

In a further aspect, the present invention relates to a pharmaceutical composition comprising the ensifentrine form A crystal or particles comprising the same as decribed above and at least one additional pharmaceutical acceptable excipient.

Preferably, the predetermined and/or effective amount of the ensifentrine form A *crystal* of the present invention is in the range of from 0.5 to 10 mg calculated as ensifentrine. For example the predetermined and/or effective amount of the ensifentrine Form A crystal of the present invention is 1 mg, 2 mg, 3 mg, 5 mg, 6 mg or 10 mg, preferably 2 mg, 3 mg or 5 mg and most preferably 3 mg calculated as ensifentrine.

In a further aspect, the present invention relates to a pharmaceutical composition suitable for administration by inhalation comprising the ensifentrine form A crystal or particles comprising the same as decribed above and at least one additional pharmaceutical acceptable excipient. The acceptable excipient is but is not limited to one or more diluents, one or more surfactants, one or more buffers or one or more tonicity adjusters.

In yet a further aspect, the present invention relates to the use of the ensifentrine Form A crystal or particles comprising the same as defined in any one of the aspects and their corresponding embodiments described in the application for the preparation of a pharmaceutical composition suitable for administration by inhalation.

In a prefered aspect, the invention relates to a pharmaceutical composition suitable for administration by inhalation comprising a liquid diluent. For instance, the diluent is water.

A liquid pharmaceutical composition according to the invention typically comprises:
(a) the particles comprising crystalline Ensifentrine Form A of the present invention at a concentration of 0.1 mg/mL to 40 mg/mL;
(b) a polyoxyethylene glycol sorbitan alkyl ester at a concentration of from 0.1 mg/mL to 2 mg/mL;
(c) a sorbitan alkyl ester at a concentration of from 0.01 mg/mL to 0.1 mg/mL;
(d) a first phosphate buffer component at a concentration of from 5 mg/mL to 8 mg/mL;
(e) a second phosphate buffer component at a concentration of from 5 mg/mL to 8 mg/mL; and
(f) a tonicity adjuster at a concentration of from 2.5 mg/mL to 8 mg/mL.

In another aspect, the present invention relates to a nebulizer comprising the pharmaceutical composition suitable for administration by inhalation decribed above.

In another prefered aspect, the invention relates to a pharmaceutical composition suitable for administration by inhalation comprising a solid diluent functioning as carrier also termed as dry powder composition. For instance, the solid diluent comprises lactose particles.

In further prefered aspect, the invention relates to a dry powder pharmaceutical composition suitable for administration by inhalation comprising: (i) particles comprising crystalline ensifentrine Form A ; (ii) coarse lactose particles having a Dv50 of from 40 µm to 80 µm; and (iii) fine lactose particles having a Dv50 of from 5 µm to 10 mm, wherein the fine lactose particles are present in an amount of from 0.2 wt% to 6.0 wt% relative to the total weight to the dry powder pharmaceutical composition.

A dry powder composition, according to the invention typically comprises:
(a) the particles comprising crystalline ensifentrine Form A at a concentration of from 0.1 weight% to 20 weight% relative to the total weight of the dry powder pharmaceutical composition
(b) coarse lactose particles at a concentration of from 80 weight% to 97 weight% relative to the total weight of the dry powder pharmaceutical composition.
(c) fine lactose particles at a concentration of from 0.5 wt% to 5.0 weight% relative to the total weight of the dry powder pharmaceutical composition.

In yet a further aspect, the present invention relates to the use of the ensifentrine Form A crystal or particles comprising the same as defined in any one of the aspects and their corresponding embodiments described above for the preparation of a pharmaceutical composition suitable for oral administration.

Preferably, the predetermined and/or effective amount of the ensifentrine form A *crystal* of the present invention is in the range of from 0.5 to 10 mg calculated as ensifentrine. For example the predetermined and/or effective amount of the ensifentrine Form A crystal of the present invention is 1 mg, 2 mg, 3 mg, 5 mg, 6 mg or 10 mg, preferably 2 mg, 3 mg or 5 mg and most preferably 3 mg calculated as ensifentrine.

The at least one pharmaceutically acceptable excipient, which is comprised in the pharmaceutical composition of the present invention, is preferably selected from the group consisting of fillers, diluents, binders, disintegrants, lubricants, glidants and combinations thereof. Preferably, the at least one pharmaceutically acceptable excipient is selected from the group consisting of lactose monohydrate, microcrystalline cellulose, povidone, croscarmellose sodium, magnesium stearate and combinations thereof. Even more preferably, all of these pharmaceutically acceptable excipients are comprised by the pharmaceutical composition of the present invention.

In a particular embodiment, the pharmaceutical composition of the present invention as describe above is a tablet, preferably a film-coated tablet comprising a tablet core and a coating, most preferably a enteric-coated tablet.

The tablet or tablet core may be prepared by mixing the crytalline ensifentrine Form A with at least one excipient such as fillers, diluents, binders, disintegrants, lubricants, glidants or combinations thereof and optionally with at least one photostabilizing agent followed by compressing the mixture. Optionally, a dry granulation step is performed before compression. Preferably, the tablet core is subsequently coated with a film-coat, whereat non-limiting examples of coatings include polyvinylalcohol-based, hydroxyethylcellulose, hydroxypropylmethylcellulose, carboxymethylcellulose sodium polyethylene glycol 4000 and cellulose acetate phthalate coatings. Methods of preparing such tablets, tablet cores, film-coated tablets and enteric-coated tablets are well known in the pharmaceutical arts.

In another particular embodiment, the pharmaceutical composition of the present invention as describe above is a capsule. In a further embodiment, the capsule shell is a gelatin shell or a hydroxypropylmethylcellulose (HPMC) shell.

In a further aspect, the present invention relates to the crystaline ensifentrine Form A, the particle comprising the crystalline ensifentrine Form A or the pharmaceutical composition comprising the crystalline ensifentrine Form A as defined in any one of the above described aspects and their corresponding embodiments for use as a medicament.

In yet another aspect, the present invention relates to the ensifentrine Form A crystal, the particle comprising the crystalline ensifentrine Form A or the pharmaceutical composition comprising the crystalline ensifentrine Form A as defined in any one of the above described aspects and their corresponding embodiments for use in the treatment and/or prevention of anemia. For example, anemia is selected from the group consisting of asthma, allergic asthma, hay fever, allergic rhinitis, bronchitis, emphysema, bronchiectasis, chronic obstructive pulmonary disease (COPD), adult respiratory distress syndrome (ARDS), steroid resistant asthma, severe asthma, paediatric asthma, cystic fibrosis, lung fibrosis, pulmonary fibrosis, interstitial lung disease, skin disorders, atopic dermatitis, psoriasis, ocular inflammation, cerebral ischaemia, inflammatory diseases, auto-immune diseases and symptoms of a COVID-19 infection.

In a particular preferred embodiment, the invention relates to the crystalline ensifentrine Form A, the particle comprising the crystalline ensifentrine Form A or the pharmaceutical composition comprising the crystalline ensifentrine Form A as defined in any one of the above described aspects and their corresponding embodiments for use in the treatment and/or prophylaxis of asthma, chronic obstructive pulmonary disease (COPD), cystic fibrosis and symptoms of a COVID-19 infection.

In another preferred embodiment, the invention concerns a method of treating and/or preventing asthma, chronic obstructive pulmonary disease (COPD), cystic fibrosis and COVID-19 infection, said method comprising administering an effective amount of the crystalline ensifentrine Form A as defined in the above described aspect and its corresponding embodiments to a patient in need of such a treatment.

Pharmaceutical compositions comprising ensifentrine and their use have been disclosed in WO 2014/140647 A1, WO 2014/140648 A1, WO 2015/173551 A1, WO 2016/042313 A1, WO 2020/074894 A1, WO 2021/028679 A1 and WO 2021/171034 A1 and are thereby incorporated as reference.

### EXAMPLES

The following non-limiting examples are illustrative for the disclosure and are not to be construed as to be in any way limiting for the scope of the invention.

### Example 1: Preparation of the ensifentrine Form A crystal of the present invention

Ensifentrine (2.0 g, e.g. prepared according to the method disclosed in example 1 of WO 2000/058308 A1) were dissolved at room temperature in a mixture of methanol (40 mL) and dichloromethane (400 mL). After one hour the solvent was removed using a rotary evaporator. The crystalline solid obtained was collected and dried at 45 °C under vacuum (25 mbar) to obtain 1.8 g (yield: 67% of theory) of the ensifentrine Form A crystal according to the present invention.

### Example 2: Powder X-ray diffraction

The ensifentrine Form A crystalline form according to the present invention was investigated by powder X-ray diffraction, which was performed with a PANalytical X'Pert PRO diffractometer equipped with a theta/theta coupled goniometer in transmission geometry, Cu-Kalpha_{1,2} radiation (wavelength 0.15419 nm) with a focusing mirror and a solid state PIXcel detector. Diffractograms were recorded at a tube voltage of 45 kV and a tube current of 40 mA, applying a stepsize of 0.013° 2-theta with 40s per step (255 channels) in the angular range of 2° to 40° 2-Theta at ambient conditions. A typical precision of the 2-Theta values is in the range of ± 0.2° 2-Theta, preferably of ± 0.1° 2-Theta. Thus, the diffraction peak of the ensifentrine Form A crystal of the present invention at 5.7° 2-Theta can appear in the range of from 5.5 to 5.9° 2-Theta, preferably in the range of from 5.6 to 5.8° 2-Theta on most X-ray diffractometers under standard conditions.

A representative diffractogram of the ensifentrine Form A crystal according to the present invention is displayed in figure 1 and the corresponding reflection list (peak list) from 2 to 30° 2-Theta is provided in table 1 below.

**Table 1: Reflection (peak) positions of the ensifentrine form A crystal according to the present invention in the range of from 2 to 30° 2-Theta; A typical precision of the 2-Theta values is in the range of ± 0.2° 2-Theta, preferably of ± 0.1° 2-Theta.**

| **Reflection position [° 2-Theta]** | **Reflection position [° 2-Theta]** | **Reflection position [° 2-Theta]** | **Reflection position [° 2-Theta]** |
|---|---|---|---|
| 5.7 | 14.2 | 21.1 | 25.6 |
| 6.7 | 16.4 | 22.2 | 26.1 |
| 8.2 | 18.7 | 22.7 | 26.4 |
| 9.2 | 19.4 | 23.3 | 27.3 |
| 9.8 | 20.0 | 23.9 | 27.9 |
| 13.5 | 20.4 | 24.7 | 28.8 |
| 13.7 | 20.8 | 25.3 | |

### Example 3: Fourier transform infrared spectroscopy

The crystalline ensifentrine Form A according to the present invention was investigated by FTIR spectroscopy. The FTIR spectrum was recorded on an MKII Golden Gate^{™} Single Reflection Diamond ATR cell with a Bruker Tensor 27 FTIR spectrometer with 4 cm⁻¹ resolution at RT. To record a spectrum a spatula tip of the sample was applied to the surface of the diamond in powder form. Then the sample was pressed onto the diamond with a sapphire anvil and the spectrum was recorded. A spectrum of the clean diamond was used as background spectrum. A typical precision of the wavenumber values is in the range of from about ± 2 cm⁻¹. Thus, the infrared peak of the ensifentrine Form A crystal according to the present invention at 3468 cm⁻¹ can appear between 3466 and 3470 cm⁻¹ on most infrared spectrometers under standard conditions.

A representative FTIR spectrum of the ensifentrine Form A *crystal* according to the present invention is displayed in figure 2 and the corresponding peak list is provided in table 2 below.

**Table 2: FTIR peak list of the ensifentrine Form A crystal according to the present invention; a typical precision of the wavenumbers is in the range of ± 2 cm⁻¹.**

| **Wavenumber [cm⁻¹]** | **Wavenumber [cm⁻¹]** | **Wavenumber [cm⁻¹]** | **Wavenumber [cm⁻¹]** |
|---|---|---|---|
| 3468 | 1594 | 1272 | 993 |
| 3351 | 1512 | 1248 | 858 |
| 3005 | 1476 | 1222 | 843 |
| 2960 | 1436 | 1202 | 814 |
| 2931 | 1402 | 1159 | 792 |
| 2852 | 1359 | 1115 | 753 |
| 1681 | 1339 | 1052 | 689 |
| 1644 | 1316 | 1037 | |

### Example 4: Differential scanning calorimetry (DSC)

The ensifentrine Form A crystal according to the present invention was investigated by DSC, which was performed on a Mettler Polymer DSC R instrument. The sample (2.72 mg) was heated in a 40 microliter aluminium pan with a pierced aluminium lid from 25 to 300 °C at a rate of 10 K/min. Nitrogen (purge rate 50 mL/min) was used as purge gas.

The DSC curve shows a first endothermic peak with an onset temperature of about 201 °C and a peak temperature of about 208 °C, and a second endothermic peak with an onset temperature of about 221 °C and a peak temperature of about 231 °C which is due to the melting of the sample. Despite the solvated nature of the crystal it shows an excellent thermal stability.

### Example 5: Thermogravimetric analysis (TGA)

The ensifentrine Form A crystal according to the present invention was investigated by TGA, which was performed on a Mettler TGA/DSC 1 instrument. The sample (16.28 mg) was heated in a 100 microliter aluminum pan closed with an aluminum lid. The lid was automatically pierced at the beginning of the measurement. The sample was heated from 25 to 300 °C at a rate of 10 K/min. Nitrogen (purge rate 50 mL/min) was used as purge gas.

The TGA curve shows no significant mass loss until the sample melts. For example mass losses of only about 0.1 weight% up to a temperature of about 180 °C, about 0.2 weight% up to a temperature of about 190 °C and about 0.3 weight% up to a temperature of about 200 °C were observed, which further proves the stability of the ensifentrine Form A crystal.

### Example 6: Gravimetric Moisture Sorption (GMS)

Moisture sorption isotherms were recorded with an SPSx-1µ moisture sorption analyzer (ProUmid, Ulm). The measurement cycle was started at ambient relative humidity (RH) of 40%. Relative humidity was then decreased to 5% RH in 5% steps, followed by a further decrease to 3% RH and to 0% RH. Afterwards RH was increased from 0% to 90% RH in a sorption cycle and decreased to 0 % in a desorption cycle in 5% steps. Finally, RH was increased from 0 to 40% RH in 5% steps.

## Claims

1. A crystalline form of ensifentrine free base (Form A) **characterized by** having a powder X-ray diffractogram comprising reflections at 2-Theta angles of (5.7 ± 0.2)°, (7.2 ± 0.2)° and (20.0 ± 0.2)°, when measured at a temperature in the range of from 20 to 30 °C with Cu-Kalpha_{1,2} radiation having a wavelength of 0.15419 nm.

2. The crystalline form according to any one of the preceding claims **characterized by** having a Fourier transform infrared spectrum comprising peaks at wavenumbers of (3468 ± 2) cm⁻¹, (2931 ± 2) cm⁻¹ and (1644 ± 2) cm⁻¹, when measured at a temperature in the range of from 20 to 30 °C with a diamond attenuated total reflection cell.

3. A particle comprising the crystal as defined in any one of the preceding claims **characterized by** having a particle size distribution with a Dv50 value of from about 0.25 µm to about 5 µm.

4. A pharmaceutical composition comprising the crystal as defined in any one of claims 1 to 3 or the particle as defined in claim 4 and at least one pharmaceutically acceptable excipient.

5. The pharmaceutical composition according of claim 5, wherein the pharmaceutical composition is suitable for administration by inhalation.

6. The pharmaceutical composition according of claim 6, wherein the pharmaceutical composition comprises a liquid diluent.

7. The pharmaceutical composition according of claim 6, wherein the pharmaceutical composition comprises a solid diluent functioning as a carrier.

8. A nebulizer comprising the pharmaceutical composition suitable for administration by inhalation according of claim 7.

9. A dry powder inhaler comprising the pharmaceutical composition suitable for administration by inhalation according of claim 8.

10. The crystal as defined in any one of claims 1 to 3, the particle as defined in claim 4 or the pharmaceutical composition according to any one of claims 5 to 8

11. Use of the crystal as defined in any one of claims 1 to 3 and the particle as defined in claim 4 for the preparation of a pharmaceutical composition.

12. The crystal as defined in any one of claims 1 to 3, the particle as defined in claim 4 or the composition as defined in any one of claims 5 to 8 for use as a medicament.

13. The crystal as defined in any one of claims 1 to 3, the particle as defined in claim 4 or the pharmaceutical composition as defined in any one of claims 5 to 8 for use in the treatment and/or prophylaxis of a condition selected from asthma, allergic asthma, hay fever, allergic rhinitis, bronchitis, emphysema, bronchiectasis, chronic obstructive pulmonary disease (COPD), adult respiratory distress syndrome (ARDS), steroid resistant asthma, severe asthma, paediatric asthma, cystic fibrosis, lung fibrosis, pulmonary fibrosis, interstitial lung disease, skin disorders, atopic dermatitis, psoriasis, ocular inflammation, cerebral ischaemia, inflammatory diseases, auto-immune diseases, and symptoms of a COVID-19 infection.

14. The use as defined in claim 14 wherein the condition is selected from asthma, chronic obstructive pulmonary disease (COPD), cystic fibrosis and symptoms of a COVID-19 infection.
